# EUROPEAN PATENT SPECIFICATION

(11) **EP 3 227 676 B1**
(45) Date of publication and mention of the grant of the patent: **31.10.2018**
(21) Application number: 15812886.8
(22) Date of filing: 27.11.2015
(51) Int. Cl.: G01N 33/18, G01N 27/66

(54) **PHOTOIONIZATION DETECTOR SYSTEM FOR ORGANICS IN WATER**
PHOTOIONISATIONSDETEKTORSYSTEM FÜR ORGANISCHE STOFFE IN WASSER
SYSTÈME DE DÉTECTION PAR PHOTO-IONISATION POUR MATIÈRES ORGANIQUES PRÉSENTES DANS L'EAU

(30) Priority: 01.12.2014 GB 201421306
(43) Date of publication of application: 11.10.2017
(73) Proprietor: Ion Science Limited, Cambridge, Cambridgeshire SG8 7UJ (GB)
(72) Inventor: DEAN, William, Francis, Cambridge Cambridgeshire CB1 2LG (GB); WITTY, Andrew, Hertford Hertfordshire SG13 7EU (GB)
(74) Representative: Harrison IP Limited
(86) International application number: PCT/IB2015/059177
(87) International publication number: WO 2016/088008

(56) References cited:
- DE-U1-202004 000 570
- US-A- 5 979 219
- US-A1- 2009 084 157
- US-B1- 8 383 046

## Description

### Field of the Invention

This invention relates to detection of gaseous and volatile analytes dissolved in water and other aqueous fluids using a detector within which such materials are sensed in the gaseous phase. Analytes of particular interest are volatile organic compounds (VOCs) particularly as found in water and aqueous solutions in concentrations varying from less than a part per billion (ppb, 10⁻⁹) to parts per thousand (ppt, 10⁻³) by mass.

### Background of the Invention

There is a need to detect and measure the presence of organic contaminants in natural and artificially contained sources of water and other aqueous fluids. VOCs include irritants, oestrogens, carcinogens and other chemicals harmful to humans, animals and plant life. Rigorous standards are laid down for potable water supplies which must be carefully monitored. Similarly, open water such as rivers, lakes and the oceans must be monitored for contamination or pollution to prevent damage to plants, animals or adjoining land.

Volatile and gaseous compounds dissolved in water are also of interest as analytes due to other deleterious effects, for example in accelerating corrosion of metal pipes and vessels, or simply as indicators of water process quality within certain environments, for example wash water used in an industrial closed loop process or in the detection of alcohols due to fermentation in food stuffs and in brewing.

It is frequently of interest to search for the presence of gaseous species within large expanses of water, and to trace their source and extent in near real time, as for example may arise from spillage of petrochemicals into sea water arising due to a natural event, an accident or clandestine activity. Such bodies of water are also subject to currents and drift. Current methods of analysis for trace quantities of VOCs and other dissolved gaseous species, such as chromatography or spectroscopy, are too slow and expensive to provide a dynamic map of volatile concentrations within water. Instead, it is preferable to submerge sensors for the target analytes within the body of water to provide a fast and quantitative measurement of their concentration. Such sensors may not provide the most accurate measure of target analytes, indeed, they may often be of most service in screening for unusual concentrations of a suite of different analytes in water samples, which are then selectively forwarded for more expensive and time consuming detailed analysis.

A class of such detectors contain a membrane, typically hydrophobic, providing a barrier between the aqueous sensed environment and a detector enclosure, through which the analyte is able to diffuse.

In one subclass of such detector, the detector enclosure contains an aqueous liquid. A well known example of this type of sensor is the Clarke electrode', in which dissolved oxygen in water diffuses from the test environment through a sensor membrane into an electrolyte, contained within a sensor cavity which includes the membrane as one containment wall member. The dissolved oxygen is consumed at an electrode proximal to the membrane. Patent DE20300514 U1, Achim Rappl, provides another example of this type of detector, again for detecting dissolved oxygen.

In another subclass of such detectors, the detector membrane, which is in contact with the aqueous fluid on one side, forms the wall of a gaseous enclosure on the other, through which the analyte is able to diffuse. Hereinafter for convenience we shall refer to this type of detector as a gas partitioned volatile detector.

US patent 5,979,219, S Sellmer-Wilsberg et al. describes such a detector in which the membrane admits volatiles, which are then entrained within a carrier gas to a nearby gas sensor which is able to respond to a volatile such as ethanol. The carrier gas is needful to ensure that analyte entering the detector can be removed within a reasonable time frame. The need for carrier gas does however constrain the application of the device to circumstances in which a carrier gas flow can be supported. The carrier gas also acts as an analyte diluent, whereupon the sensitivity of a device provided by this invention is limited and its accuracy circumscribed by the extent of gas flow through the membrane.

US 5,979,219 describes another gas partitioned volatile detector in which the membrane admits volatiles such as ethanol into the sensing gaseous enclosure which is then sensed by a proximal semiconducting gas sensor. The invention relies on a pressure relief system being continuously open in order to 'exhaust' gases, not least target volatiles, into the atmosphere [Col 2 lines 11-32, Col 3,lines 26-35] rendering a carrier gas to flush the sensor enclosure unnecessary [Col 2 line 31]. A problem to be expected with a detector made according to US '219 is that the rate of analyte escape from the detector gaseous chamber will be very slow and highly variable, according to factors which enable more or less rapid escape of gases from the pressure relief outlet to atmosphere at some considerable distance. The design may be sufficiently compact to enable detector response and cleardown in the time frame of half a day, as may be useful in brewing, but not within a minute or two, as may be required, for example, in sensing volatiles in a sensor train behind a boat or in a water process stream.

US 7,385,191 B1, assigned to Pacific Environmental Technologies, LLC, provides another illustration of a gas partitioned volatile detector in which a much more rapid response time is assured by the continuous vacuum purge required for the mass spectrometer sensing gases permeating the gas-permeable membrane. It is however, liable to be limited by the burden of cost and care that is need to maintain a vacuum system, not to mention practicalities of its being used in all weathers by persons who may have limited training in the use of vacuum systems.

In the absence of an air purge or a gas release means or a vacuum system, a further means of analyte removal from a gas partitioned volatile detector may be provided by the gas sensor or gas sensing element itself consuming the analyte, as is illustrated in EP0734516A1, Commonwealth Scientific and Industrial Research Organisation, which describes an amperommetric oxygen sensor behind a diffusive membrane.

Analyte detection may be contemplated by way of infrared sensors such as disclosed in US 8,383,046 or by humidity sensors such as disclosed in WO2006/042546. Most prior art to date does not appear to include a gas partitioned volatile detector in which neither does the gas sensing element consume the analyte, nor does the detector use some means of gas evacuation to remove the analyte. One exception to this is US 8,383,046 which utilises an IR detector typically having a volume of 400mm³. This technology is dependent on having a relatively large volume sample in order to register the presence of the analyte. The downside of this is that the time taken to fill the chamber (by equilibration of analyte across a sensor membrane) to an appreciable level for a reading to be taken makes for a sensor whose response time is unacceptably high.

The present invention recognises this and all previously described problems and attempts to mitigate them by way of a novel detector arrangement. Hereinafter for convenience we will refer to this type of detector as a gas-equilibrated, volatile-in-liquid detector.

This detector provides a significant advantage over other gas-partitioned volatile detectors, in prospectively delivering a partial pressure of gas in the gaseous enclosure *p* in equilibrium with the concentration of gas in the liquid phase, *c.* When this condition is achieved, Henry's Law is obeyed. Specifically, *p* = *k*_{H} x *c*, *where k*_{H} is Henry's constant. Temperature dependent constants *k*_{H} are known for a plethora of important volatiles and thus, prospectively, the response of a gas sensing element can be directly related to the concentration of the gas in the liquid phase. Thus, it requires only the gas sensing element in the gas-equilibrated, volatile-in-liquid detector to be reliable in sensing a target analyte for the concentration of a volatile in an adjoining liquid phase to be known. The detector can provide a measurement of a volatile in a liquid which does not vary directly with the rate of supply of analyte to the sensor, as determined either: by the rate of consumption or dispersion of analyte by components within the detector; or by the permeability or porosity of any intervening membrane.

A possible reason why the prior art may not have been directed towards gas-equilibrated, volatile-in-liquid detectors is that in order to achieve gas/liquid equilibrium, particularly for volatile analytes for which *k*_{H} is large and for which accordingly the volatile is only scarcely soluble in water, the volatile must be abstracted in substantial quantity from the sampled aqueous phase, and that that this volumetric demand for analyte on the sample side increases in proportion to the volume of the gaseous enclosure containing the gas sensing element. The gaseous enclosure within a gas-equilibrated, volatile-in-liquid detector is therefore required to be smaller than is demanded commonly of atmospheric gas sensing devices monitoring. Unlike other detectors engaging a water-gas partition, such as those of US 5979219, US 7385191 B1, DE20300514 U1 and EP0734516A1 referred to above, the effectiveness of this type of detector requires the elimination of all means of removal of gas from the gaseous enclosure within the sensor except from the membrane itself. Achieving this condition is not trivial.

Another problem we have encountered in the discovery of the present invention is that gas sensors and gas sensing sub-assemblies suitable for the measurement of analyte in equilibrium with an adjoining liquid phase commonly include a gaseous enclosure characterised by its being contiguous, sometimes unavoidably, with tortuous and convoluted gaseous spaces between sub-assembled components and with capillary leak sites, all of which provide an appreciable sink for analyte when compared to the slow rate of supply and removal of analyte though the partition separating the gas enclosure from the liquid containing the analyte.

A further difficulty arises from the need to obtain a sufficient flux through the membrane separating an aqueous environment from a gaseous enclosure to ensure reasonably fast equilibration of the analyte within the gaseous enclosure. To obtain this, it is commonly preferable to engage a porous hydrophobic membrane as a partition between the liquid and the detector's gaseous enclosure. Placement and sealing of the membrane is problematic. Placement and seal at some separation from the gas sensor or sensing sub-assembly within the detector requires an intervening membrane support, to ensure the membrane does not rupture under a pressure presented by the liquid abutting its external face, as taught for example by US 5979219. Remoteness of the membrane from the gas sensing element in an equilibrated detector adds significantly to the time required for equilibration of gas within an equilibrated detector. If the gaseous enclosure members are used to support and seal the membrane, without the use of an o-ring, and contrary to the normal practice in the art as to how such liquid/gas seals should be assured, then any membrane rupture or leakage will lead to ingress of liquid into the gaseous enclosure, prospectively proceeding to corrode electrically connected gaseous sensing components and causing the sensor to be worthless.

### Summary of the Invention

We have invented a photoionisation (PID) detector sensor head according to claim 1 capable of detecting and measuring organic materials in water, particularly at trace concentrations, which can provide real time measurements in a field environment, with little additional procedure than is required in the use of a typical gas detectors, and which enables easily calculated determination of the concentration of such organic materials in water, when their identity is known.

According to the present invention, a photoionisation detector (PID) sensor head is provided for use in a detector of volatiles in aqueous media as set forth in claim 1 of the appended claims.

Thus, we provide a detector of volatiles in water and aqueous fluids, which contains as one member a vapour-porous or vapour-permeable membrane forming a wall of a gaseous enclosure in which concentration of gas in the enclosure is substantively achieved by means of analyte flux through the membrane.

The membrane may also be sealed to an external wall of the gas-sensing chamber or of a surrounding enclosure, which may form part of a separable gas sensing sub-assembly or easily removable sensor. This outer seal may be a peelable adhesive. Conveniently, the two seals are concentric.

In this configuration, the vapour-porous or vapour-permeable membrane forms an entry point to a second gaseous enclosure external of the gas-sensing chamber. This can reduce the effect of leaks between sub-components of the sensor assembly, by enabling gas equilibration in the space between the gas sensor and walls containing it.

It is preferable for the membrane sealing the gas sensing sub-assembly or gas sensor to extend beyond its point of sealing to the sensor to affect a further seal to outer detector components, such that, upon removal of the membrane, the gas sensor or sensor sub-assembly may be removed from the detector. To facilitate this, it is convenient for the gas sensor or gas sensing sub-assembly to receive gas from an orifice in a planar supporting surface, which is contrived to be in the plane of the termination of detector wall members to which the outer membrane seal is made.

It is self-evident that, as far as possible, the membrane must exclude liquid water from the gas-sensing chamber. Conveniently, the membrane is hydrophobic. A suitable material may be porous polytetrafluoroethylene (PTFE) or polyvinylidene difluoride (PVDF), either stand alone or as a coating on a porous substrate such as polypropylene. Typically, the overall thickness is some 100 - 500 m⁻⁶ and the pore size is approximately 0.5 m⁻⁶.

The seal to the outer membrane member may be effected by means of an adhesive layer on a hydrophobic surface such as PTFE.

The membrane may contain an annulus of adhesive to effect sealing of the gas sensor or gas sensing subcomponent.

Ideally, the seal to the periphery of the chamber orifice is a vapour-impermeable barrier throughout the thickness of the membrane. This is normally a weld, particularly ultrasonic, between the membrane material and the gas sensor or gas sensing sub-assembly but can also be an impermeable adhesive penetrating the membrane.

The invention in a further aspect provides for membrane material between the outer membrane to detector wall seal and the inner gas membrane to gaseous sensor seal or membrane to gaseous sensing sub-assembly seal to be vapour-permeable or vapour-porous.

It is preferable for all porous surfaces to comprise porous PTFE.

Without limitation, it is believed that the vapour detector measures the partial pressure of the organic materials in the vapour phase. Particularly at low concentrations, this measurement is a function of the concentration of the organic material in the liquid water.

Advantageously, the gas-sensing chamber is so configured as to minimise dead volume therein. Conveniently, according to the invention the internal volume of the gas-sensing chamber is no greater than 100 mm³.

Advantageously, the electrodes or other sensing system components within a sensor or sensing sub-assembly according to the present invention are stacked, with only a modest spacing between the sensing components such that the entire thickness of the sensing enclosure is no greater than a few millimetres. Thereby, the water from which organic volatiles pass through the membrane is in very close proximity to the entirety of the gas sensing enclosure, typically within 2 mm. The sensor may include a plurality of electrode contacts which, in an assembled position, lie in a common plane in the gas-sensing chamber and as close as possible to the overlying membrane.

A particularly beneficial stacking arrangement for the electrodes is described and claimed in our patent GB 2449664 B, filed 30 June 2007 (equivalent to US 7821270 B2 issued 26 October 2010 and others elsewhere). The electrode stack of this patent is well-suited to use with a miniature photoionisation detector.

### Brief Description of the Drawings

Figure 1 shows a schematic representation of a gas-equilibrated, volatile-in-water detector not within the scope of the invention.
Figure 2 is an elevation of the membrane assembly of Figure 1 prior to its deployment.
Figure 3 shows the membrane assembly of Figure 2 from a different perspective.
Figure 4 shows a schematic representation of a gas-equilibrated, volatile-in-water probe according to the invention wherein a gas sensor body contains a removable and replaceable gas receiving and sensing member.
Figure 5 shows a gas sensing replaceable sub-assembly prior to assembly in the probe shown schematically in Figure 4.

### Description of Embodiments

The invention will now be described in more detail by reference to the drawings provided.

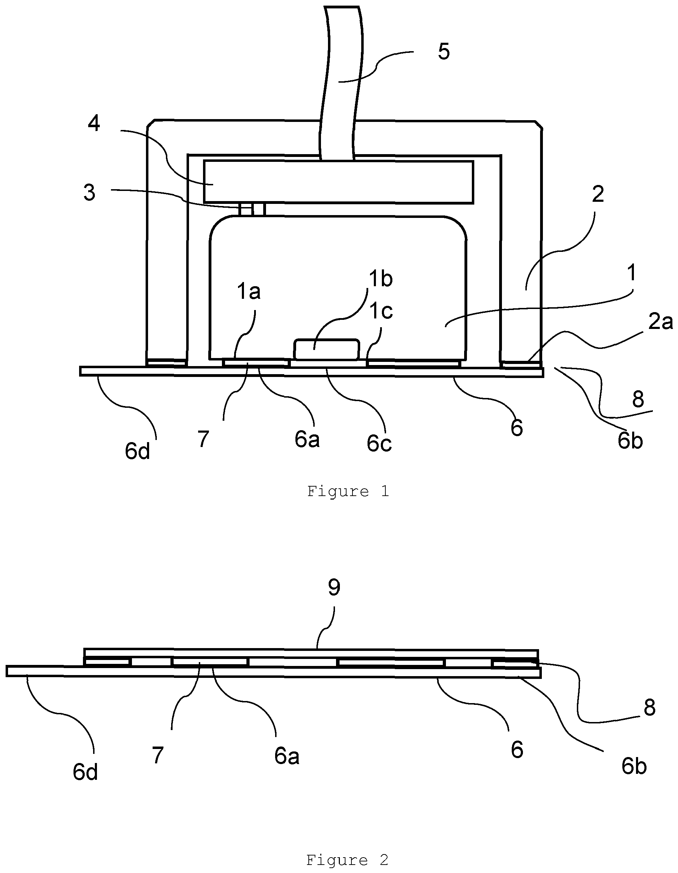

Figure 1 shows a schematic representation of a gas-equilibrated, volatile-in-water detector, in which a gas sensor 1 is contained within a gas sensor cradle 2. The gas sensor is preferably of cylindrical shape, and typically of 20 mm diameter and 16.6 mm height, excluding contact pins 3, as provided as a received industrial standard by gas sensor manufacturers such as Alphasense Ltd, Dynament Ltd and City Technology Ltd. The gas sensor preferably is disposed to sit upon, and make electrical connection via 3 to, a small printed circuit board (PCB), 4, itself electrically connected to a cable 5, through which power is delivered to the gas sensor and signals are communicated to and from the sensor to some external means of control such as a computer, via, by way of example, a universal serial port. The cradle 2 and PCB 4 are positioned so that the face, 1a, of the gas sensor containing a gas sensing orifice 1b, is approximately co-planar with the cradle wall flat surfaces 2a. Gas porous PTFE membrane 6, some 280 - 300 m⁻⁶ thick, is attached by means of a thin adhesive layer 7 to the sensor face 1a. A further portion 6b of the membrane is attached by means of a thin layer of adhesive 8 to the cradle wall flats 2a, thereby forming an entry point to a second gaseous enclosure external of the gas sensor 1. The adhesive on the membrane does not extend beyond its point of contact to the gas sensor face 1a at the periphery of the gas sensing orifice 1c. Over at least its area of coverage of the gas orifice, the membrane portion 6c is on the contrary, substantially free of adhesives which could prevent gas flow into or out of the gas orifice. There may be an additional membrane component 6d, which extends beyond cradle wall segments 2a, enabling the membrane to be peeled off after field use, rather than being stored, possibly wet and contaminated, attached to other detector members.

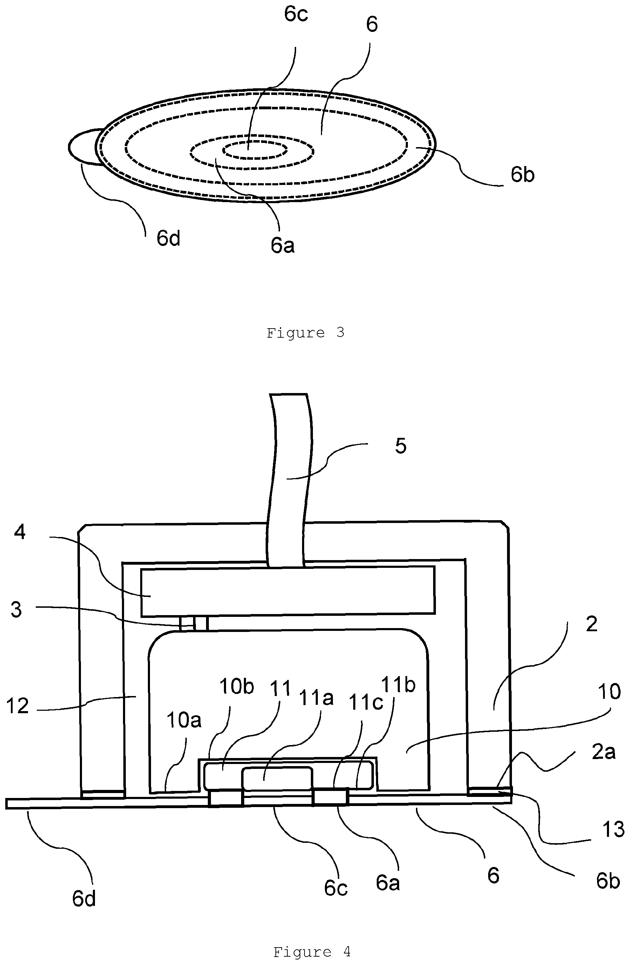

Figure 2 shows the membrane assembly of Figure 1 prior to its deployment. Additional to the components already described, the membrane includes a waxed paper or other removable member 9 to protect adhesive portions of the membrane before use. Figure 3 shows the membrane assembly of Figure 2 from a different perspective, with the adhesive-protective element removed.

Figure 4 shows a schematic representation of a gas-equilibrated, volatile-in-water probe provided by the invention wherein a gas sensor body 10 contains a removable and replaceable gas receiving and sensing member 11. An example of such a sensor is the 16.6 mm x 20mm diameter miniature photoionisation detector (PID) manufactured by Ion Science Limited and containing an electrode pellet as described and claimed in our GB 2449664 B.

Sensor body 10 is seated on PCB 4, with which it makes electrical contact by means of pins 3. PCB 4 is also in electrical contact with cable 5, as described in reference to corresponding components 3, 4 and 5 in Figure 1.

Gas sensing member 11 is approximately pellet shaped, and includes an orifice for receiving gas, 11a, on its outwardly facing major surface, 11b, opposing its other major surface proximal to the sensor body cavity (gas sensing chamber) 10b containing the gas sensing member 11. Surface 11b of the gas sensing member 11 is approximately co-planar with sensor body surface 10a, and also approximately co-planar with cradle wall flats 2a.

Gas sensing member 11 is attached at annulus 11c to gas-porous or gas-permeable membrane 6 by means of an adhesive or ultrasonic welding of membrane portion 6a to the gas sensing member flat surface 11b close to the periphery of gas orifice 11a. It is preferable for the membrane portion 6a joined to gas sensing member portion 11c not to be porous or permeable to gas, either by virtue of an impermeable adhesive being applied to and impregnating the membrane, or by the membrane 6 and gas sensing member face 11b being welded at the point of fusion so as to form a seal that is neither porous nor appreciably permeable.

The membrane 6 is also attached to cradle wall flats 2a by means of adhesive 13 at annulus 6b, thereby forming an entry point to a second gaseous enclosure 12 external of the gas-sensing chamber. The membrane is substantially porous or vapour-permeable over that portion overlaying gas orifice 11a and bounded by the impervious seal between annulus 6a and portion 11c. The membrane portion between annular seals 6a and 6b may or may not be porous or permeable according to the benefit conferred by enabling gaseous analyte gas irrigation at potential leak paths between probe members 10 and 11. The membrane 6 further may include a tab 6d for convenient removal of the membrane 6 after use.

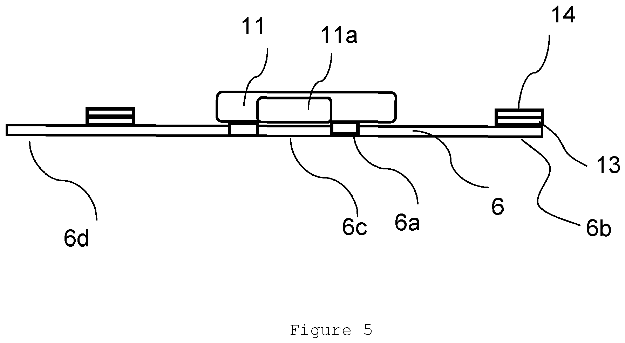

Figure 5 shows a replaceable gas sensing sub-assembly comprising components 11 and 6 prior to their assembly in the probe shown schematically in figure 4. This replaceable component includes a removable protective cover 14 to protect the adhesive portion of the membrane 6b.

The use of the sensors will now be described by reference to how they are operated in order to measure volatiles present in a watery liquid.

In the case of a gas sensor containing an integral means of gas admittance, such as is described in Figure 1, it is preferable for the sensor 1 to be removed from the cradle 2 over times of significant storage. The sensor is manually fitted in the cradle ensuring pins from the sensor 3 fit snugly into PCB platform 4. The covering 9 is removed from a disposable membrane assembly such as shown in Figure 2, and placed over the cradle wall flats 2a and sensor face 1a to make a seal. To ensure correct alignment of the membrane to the sensor face, such that a porous and permeable part of the membrane 6c overlays the gas sensing orifice 1b it is preferable for the membrane and cradle to include means of co-alignment such as notches (not shown).

The probe is now connected to a means of power supply and data communication via cable 5. The probe may be calibrated by placing a gas hood over the assembled probe, and presenting a suitable concentration of the analyte to the gas hood. Alternatively, the probe may be calibrated using an aqueous liquid, typically pure water, into which the gaseous analyte is dissolved. The former is generally preferable, and provides an advantage of using a gas-equilibrated, volatile-in-liquid detector over other volatile-in-water detection technologies. These previous detectors require aqueous reference samples, which are prone to loose the volatile as soon as their means of containment, typically a glass ampoule, is breached.

Typically the cable 5 connected to the probe is flexible and armoured or sheathed so as to provide protection when submerged in a watery fluid, perhaps under very adverse conditions. The probe may include additional members to ensure to protect the assembly from damage, particularly the probe itself. It is however deleterious for the membrane 6 to be appreciably obscured from the aqueous environment presented to it, insofar as free flow of fluids across it is needed for dynamic sensing.

The removal of the sensor may benefit from additional cradle members, not shown in Figure 1, which enable the cradle walls surrounding the circular or cylindrical section of the gas sensor to be partly dismantled.

The sensor shown in Figure 4 is best stored with its removable components removed from the sensor cradle 2. The sensor may be manually assembled as described above, although in this case the membrane assembly shown in Figure 5 is affixed to the sensor cradle 2 only after careful orientation and assembly of gas sensor sub-components. In the PID sensor, a lamp is first inserted in the gas sensing member 11 opposite the membrane orifice 11a. Then the protective backing 14 to the adhesive section is removed. Then the membrane assembly is placed over the cradle wall flats 2a as shown in Figure 4 and pressed down to ensure a water tight seal.

Following tests with the probe shown in Figure 4, it is again advisable for the probe to be washed off in clean water and dried. The membrane 6 is peeled off cradle wall flats 2a. After removal of the sensor, the entire sub-assembly shown in Figure 5 is removable and disposable, being a small, environmentally benign and modest cost item. The sensor body can then be removed and stored in a separate storage capsule or refitted with a new membrane assembly as depicted in Figure 5, ready for subsequent use.

The instrument is calibrated as described above. In the PID, the use of a gas for calibration is a particular advantage where the PID is being used to trace a volatile to which PID responds, with reasonable assurance as to the presence of that volatile. For example, it might be calibrated with 1 ppm xylene in air, using Henry's Law to determine the equivalent xylene-in-water concentration.

Possible applications of the invention include, for example, remediation of polluted water, monitoring of potable water, food and drink processing, and regulatory enforcement.

## Claims

1. A photoionisation detector (PID) sensor head for use in a detector of volatiles in aqueous media, which head includes a miniature photoionisation detector comprising a gas sensor body (10) and a replaceable gas sensing member (11), said gas sensor body (10) having a sensor body cavity defining a gas sensing chamber (10b) containing said gas sensing member (11), the head further including a vapour-porous or vapour-permeable membrane (6) forming an entry point (6c) into the otherwise closed gas-sensing chamber, **characterised in that** the internal volume of the gas-sensing chamber (10b) is no greater than 100 mm³.

2. A sensor head as claimed in claim 1, wherein the membrane (6) is sealed to the periphery of a chamber orifice (11a) by means of a chamber orifice seal (7) and forms an entry point to a second gaseous enclosure external to the gas-sensing chamber.

3. A sensor head as claimed in claim 1 or 2, wherein a surrounding enclosure seal (8) seals the membrane (6) to an external wall (2a) of the gas-sensing chamber or of a surrounding enclosure.

4. A sensor head as claimed in claim 13, wherein the external wall seal (8) seals by means of a peelable adhesive.

5. A sensor head as claimed in claim 13 or 14, wherein the two seals (7,8) are concentric.

6. A sensor head as claimed in any preceding claim depending from claim 2, wherein the gas-sensing chamber orifice (11a) is in a planar supporting surface coplanar with the termination of a wall of a surrounding enclosure (2).

7. A sensor head as claimed in any preceding claim depending from claim 2, wherein the seal (7) to the periphery of the chamber orifice (11a) is a vapour-impermeable barrier throughout the thickness of the membrane (6).

8. A sensor head as claimed in any preceding claim depending from claim 2, wherein the or each seal (7,8) is an annulus.

9. A sensor head as claimed in claims 1 to 8, wherein the membrane (6) is hydrophobic.

10. A sensor head as claimed in claims 1 to 9, which is detachable from an assembly supporting the sensor head.

11. A sensor head as claimed in any claim depending from claim 2, wherein the seal (7) to the periphery of the chamber orifice is a weld or an impermeable adhesive penetrating the membrane (6).

12. A sensor head as claimed in claims 1 to 11, wherein the sensor (10, 11) included in the sensor head includes a plurality of electrode contacts which lie in a common plane in the gas-sensing chamber.

13. A sensor head as claimed in claims 11 to 12, wherein the gas-sensing chamber (10b) is so configured as to minimise dead volume therein.

14. A sensor head as claimed in claims 1 to 13, wherein elements of the sensor are within 2mm of the membrane (6)so as to reduce the response time of the sensor.

15. A gas-equilibrated, volatile-in-liquid detector incorporating a sensor head as claimed in any one of Claims 1 to 14.

## Patentansprüche

1. Sensorkopf für einen Photoionisationsdetektor (PID) zur Verwendung in einem Detektor für flüchtige Bestandteile in einem wässrigen Medium, wobei der Kopf einen Miniatur-Photoionisationsdetektor aufweist, der einen Gassensorkörper (10) und ein austauschbares Gasmesselement (11) umfasst, wobei der Gassensorkörper (10) eine Sensorkörperkavität aufweist, die eine Gasmesskammer (10b) definiert, die das Gasmesselement (11) enthält, wobei der Kopf ferner eine Dampf-Porenmembran oder eine dampfdurchlässige Membran (6) aufweist, die eine Eintrittsstelle (6c) in die ansonsten geschlossene Gasmesskammer bildet, **dadurch gekennzeichnet, dass** das Innenvolumen der Gasmesskammer (10b) nicht größer ist als 100 mm³.

2. Sensorkopf nach Anspruch 1, wobei die Membran (6) zur Peripherie einer Kammeröffnung (11a) durch eine Kammeröffnungsdichtung (7) abgedichtet ist und eine Eintrittsstelle in eine zweite Gaseinfassung außerhalb der Gasmesskammer bildet.

3. Sensorkopf nach Anspruch 1 oder 2, wobei eine umgebende Einfassungsdichtung (8) die Membran (6) an einer Außenwand (2a) der Gasmesskammer oder einer umgebenden Einfassung abdichtet.

4. Sensorkopf nach Anspruch 13, wobei die Außenwanddichtung (8) durch ein abziehbares Adhäsionsmittel abdichtet.

5. Sensorkopf nach Anspruch 13 oder 14, wobei die beiden Dichtungen (7, 8) konzentrisch sind.

6. Sensorkopf nach einem der vorstehenden Ansprüche in Abhängigkeit von Anspruch 2, wobei sich die Gasmesskammeröffnung (11a) in einer planaren Stützoberfläche befindet, die koplanar ist mit dem Abschluss einer Wand einer umgebenden Einfassung (2).

7. Sensorkopf nach einem der vorstehenden Ansprüche in Abhängigkeit von Anspruch 2, wobei die Dichtung (7) an der Peripherie der Kammeröffnung (11a) eine dampfundurchlässige Sperre durch die Dicke der Membran (6) ist.

8. Sensorkopf nach einem der vorstehenden Ansprüche in Abhängigkeit von Anspruch 2, wobei die oder jede Dichtung (7, 8) ein Ring ist.

9. Sensorkopf nach einem der Ansprüche 1 bis 8, wobei die Membran (6) hydrophob ist.

10. Sensorkopf nach einem der Ansprüche 1 bis 9, der von einer Einheit lösbar ist, welche den Sensorkopf stützt.

11. Sensorkopf nach einem der vorstehenden Ansprüche in Abhängigkeit von Anspruch 2, wobei die Dichtung (7) an der Peripherie der Kammeröffnung eine Schweißnaht ist oder ein undurchlässiges Adhäsionsmittel, das die Membran (6) penetriert.

12. Sensorkopf nach einem der Ansprüche 1 bis 11, wobei der in dem Sensorkopf enthaltene Sensor (10, 11) eine Mehrzahl von Elektrodenkontakten aufweist, die in einer gemeinsamen Ebene in der Gasmesskammer liegen.

13. Sensorkopf nach einem der Ansprüche 1 bis 12, wobei die Gasmesskammer (10b) so gestaltet ist, dass sie das Totvolumen darin minimiert.

14. Sensorkopf nach einem der Ansprüche 1 bis 13, wobei sich Elemente des Sensors innerhalb von 2 mm von der Membran (6) befinden, um die Ansprechzeit des Sensors zu verringern.

15. Detektor mit Gasausgleich für flüchtige Bestandteile in Flüssigkeit, der einen Sensorkopf nach einem der Ansprüche 1 bis 14 aufweist.

## Revendications

1. Tête de capteur de détecteur par photo-ionisation (PID) destinée à être utilisée dans un détecteur de substances volatiles en milieu aqueux, ladite tête comprenant un détecteur par photo-ionisation miniature comprenant un corps de capteur de gaz (10) et un élément de détection de gaz remplaçable (11), ledit corps de capteur de gaz (10) ayant une cavité de corps de capteur définissant une chambre de détection de gaz (10b) contenant ledit élément de détection de gaz (11), la tête comprenant en outre une membrane poreuse ou perméable à la vapeur (6) formant un point d'entrée (6c) dans la chambre de détection de gaz autrement fermée, **caractérisée en ce que** le volume interne de la chambre de détection de gaz (10b) n'est pas supérieur à 100 mm³.

2. Tête de capteur selon la revendication 1, la membrane (6) étant scellée à la périphérie d'un orifice de chambre (11a) au moyen d'un joint (7) d'orifice de chambre et formant un point d'entrée à une seconde enceinte gazeuse externe à la chambre de détection de gaz.

3. Tête de capteur selon la revendication 1 ou 2, un joint d'enceinte périphérique (8) scellant la membrane (6) sur une paroi extérieure (2a) de la chambre de détection de gaz ou d'une enceinte périphérique.

4. Tête de capteur selon la revendication 13, le joint de paroi externe (8) scellant au moyen d'un adhésif pelable.

5. Tête de capteur selon la revendication 13 ou 14, les deux joints (7, 8) étant concentriques.

6. Tête de capteur selon l'une quelconque des revendications précédentes dépendant de la revendication 2, l'orifice de chambre de détection de gaz (11a) étant dans une surface d'appui plane coplanaire avec la terminaison d'une paroi d'une enceinte périphérique (2).

7. Tête de capteur selon l'une quelconque des revendications précédentes dépendant de la revendication 2, le joint (7) à la périphérie de l'orifice de chambre (11a) étant une barrière imperméable à la vapeur sur toute l'épaisseur de la membrane (6).

8. Tête de capteur selon l'une quelconque des revendications précédentes dépendant de la revendication 2, le ou chaque joint (7, 8) étant un anneau.

9. Tête de capteur selon l'une quelconque des revendications 1 à 8, la membrane (6) étant hydrophobe.

10. Tête de capteur selon l'une quelconque des revendications 1 à 9, qui est détachable d'un ensemble supportant la tête de capteur.

11. Tête de capteur selon l'une quelconque des revendications dépendant de la revendication 2, le joint (7) à la périphérie de l'orifice de chambre étant une soudure ou un adhésif imperméable pénétrant dans la membrane (6).

12. Tête de capteur selon l'une quelconque des revendications 1 à 11, le capteur (10, 11) compris dans la tête de capteur comprenant une pluralité de contacts d'électrodes qui se trouvent dans un plan commun dans la chambre de détection de gaz.

13. Tête de capteur selon la revendication 11 ou 12, la chambre de détection de gaz (10b) étant conçue de sorte à minimiser le volume mort en son sein.

14. Tête de capteur selon l'une quelconque des revendications 1 à 13, les éléments du capteur étant à moins de 2 mm de la membrane (6) de sorte à réduire le temps de réponse du capteur.

15. Détecteur de substance volatile dans un liquide à équilibre gazeux comprenant une tête de capteur selon l'une quelconque des revendications 1 à 14.
